# EUROPÄISCHE PATENTSCHRIFT

(11) **EP 1 669 061 B1**
(45) Veröffentlichungstag und Bekanntmachung des Hinweises auf die Patenterteilung: **18.11.2009**
(21) Anmeldenummer: 05021762.9
(22) Anmeldetag: 06.10.2005
(51) Int. Cl.: A61K 8/60, C11D 1/66, C11D 11/00

(54) **Treibgasfreie schaumbildende Systeme, deren Herstellung sowie deren kosmetische, dermatologische und hygienische Anwendungen**
Foam forming systems devoid of propellant gas, the production as well as the cosmetic, dermatological and hygienical use thereof
Systèmes sans carburant gazeux, leur préparation ainsi que leurs utilisations cosmétiques, dermatologiques et hygiéniques

(30) Priorität: 22.10.2004 DE 102004051420
(43) Veröffentlichungstag der Anmeldung: 14.06.2006
(73) Patentinhaber: Merz Pharma GmbH & Co. KGaA, 60318 Frankfurt Main (DE)
(72) Erfinder: Beutler, Rolf D., Dr., 64739 Höchst/Hummetroth (DE); Knebl, Robert, Dr., 71549 Auenwald (DE); Dogs, Carsten, 64546 Mörfelden - Walldorf (DE); Nürnberg, Eberhard, Prof. Dr., 91080 Uttenreuth-Weiher (DE); Nürnberg, Wolf, Dr., 18225 Kühlungsborn (DE); Sawiec, Joanna, 91058 Erlangen (DE)
(74) Vertreter: Müller, Claudia

(56) Entgegenhaltungen:
- DE-A1- 10 258 958
- US-A- 5 368 850
- US-A1- 2002 122 772

## Beschreibung

### Gegenstand der Erfindung

Die vorliegende Erfindung betrifft schaumbildende Systeme, welche einen Kohlenhydrat-Tensidanteil enthalten und ohne Treibmittel zur Schaumbildung befähigt sind. Die Grundlagen sind vorzugsweise hypoallergen.

Die Erfindung betrifft insbesondere flüssige Zubereitungen auf wässriger oder wässrig -alkoholischer Basis, welche spezielle Kohlenhydrat (=Zucker-) Tenside, insbesondere in Kombination mit bestimmten Co-Tensiden umfassen, die den Charakter von O/W-Tensiden aufweisen können. Diese können zur kosmetischen oder zur dermatologischen Anwendung oder auch als Desinfektionsmittel, insbesondere für medizinische oder hygienische Zwecke wie Vorrichtungen, Haut- / Handdesinfektion eingesetzt werden.

### Hintergrund der Erfindung

Bisher eingesetzte schaumbildende Produkte zur kosmetischen, pharmazeutischdermatologischen oder desinfizierenden Anwendung enthalten prinzipiell Treibgasmittel. Dabei handelt es sich insbesondere um Propan- Butan- Gasgemische, welche in einen geeigneten Behälter gefüllt werden müssen. Darüber hinaus besteht das Problem einer leichten Entflammbarkeit solcher Mittel. In verschäumbaren zweiphasigen Systemen wie Emulsionen werden darüber hinaus zur Schaumstabilisierung zusätzliche Substanzen, insbesondere W/O-Tenside, Lipide und gegebenenfalls Polymere eingesetzt. Die hierbei vorhandenen Tenside (Emulgatoren) können sensibilisierende und/oder hautreizende Wirkungen entfalten. Daher sollten vor allem offizielle, d.h. in amtlichen Arzneibüchem (Ph.Eur.) aufgeführte Substanzen mit nachgewiesenen günstigen physiologisch- dermatologisch - kosmetologischen Eigenschaften zum Einsatz kommen. Soll bei der Anwendung eine desinfizierende Wirkung erzielt werden, wird insbesondere auch Alkohol als Komponente eingesetzt. Dieser wirkt jedoch bekanntlich schaumdestabilisierend.

Wünschenswert wäre indessen ein Produkt, welches mittels Schaumbildung ausgetragen wird, und sodann durch ein kontrolliertes Kollabieren des Schaumes zu einer besonders guten Verteilung gebracht werden kann.

In der EP A 0 985 408 werden Zubereitungen mit Desinfektions- und insbesondere Desodorierungswirkung für eine kosmetische oder dermatologische Anwendung beschrieben. Diese sollen eine Wirkstoffkombination aus einem antiadhäsiven Mittel A und einem darauf abgestimmten Mikrobizid B zwecks synergistischer Steigerung des Mikrobizids B aufweisen. Eine derartige Kombination kann in Form von Gelen, Cremes, Seife etc. in den dafür bereiteten Anwendungsformen zusammen mit entsprechenden Zusatzstoffen vorliegen. Als antiadhäsive Mittel A werden allgemein solche genannt, welche für spezielle Mikroorganismen spezifisch sein sollen. Hierzu gehören z. B. Aminozucker, Saccharide, Glykolipide u. ä. Als Mikrobizide B werden u. a. Aldehyde wie Anisaldehyd, Glyceride, aromatische Carbonsäuren, Nitrile, Fettalkohole, Arylverbindungen, Terpene, halogenierte aromatische Verbindungen, Konservierungsmittel, Ceramide, Sterole, Steroidhormone und insbesondere Fettsäuren wie Ölsäure, Hexadecansäure, Azelainsäure, Zimtsäure, Glycerinmonolaurat, Diglycerinmonocaprinat, Kokosfettsäure genannt.. Bevorzugt ist die Menge an A größer als die Menge an B. So wird ein Propan-Butangasgemisch enthaltendes Aerosolspray mit Octyldodecanol (B), Laurinsäuresucroseester (A), Diglycerinmonocaprinat (B) und als Rest (ca. 93%) Ethanol beschrieben. Mit einer solchen Zusammensetzung ist, wie ersichtlich, keine treibgasfreie Schaumbildung möglich. Ohne Treibgas ist ein Rasierschaum mit Natriumlaurylsulfat als schaumbildende Komponente, Montanov 68 (Cetearyl Alkohol + Cetearylglycosid) als Komponente A (Antiadhäsivum) und Anisaldehyd als Substanz B beschrieben. Für ein Glycerinmonocaprylat, PEG 40 hydriertes Rizinusöl sowie Sucrosemyristat enthaltendes Pumpspray wird ebenfalls keine Verschäumbarkeit angegeben. Eine treibgasfreie und zugleich schaumbildende Zusammensetzung ist insofern hier nicht beschrieben. DE 102 58 958 betrifft eine Kombination aus ethoxylierten anionischen Glyceriden mit amphoteren Tensiden / Alkyl-Glycosiden sowie Ölkomponenten, zur Reinigung der Haut. DE 103 07 469 beschreibt mit einem Treibgas verschäumbare kosmetische Zusammensetzungen aus anionischen Tensiden, Decyl-,lauryl-,Kokosglycosid, sowie zwingend Glycerinmonooleat zur Reinigung der Gesichtshaut.

In der US 2002/0122772 A1 werden treibgashaltige Reinigungsmittel aus einer Tensidkombination mit anionischen sowie amphoteren und ggf. nichtionischen Tensiden beschrieben. Die US 5,368,850 offenbart wässrige Dispersionen aus einer Zuckerverbindung, einem Fettsäureester und einem Acrylamidcopolymer zur Behandlung von Haut oder Haar.

### Aufgabe vorliegender Erfindung

Aufgabe vorliegender Erfindung ist es, Zubereitungen zu entwickeln, welche eine Grundlage aufweisen, mit der eine treibgasfreie Schäumung, insbesondere auch in Gegenwart höherer Mengen Alkohol, möglich ist und welche sowohl kosmetisch dermatologisch, insbesondere topisch, als auch als desinfizierendes Mittel für Oberflächen eingesetzt werden kann und keine Unverträglichkeiten bzw. störenden Einflüsse auf die behandelte Fläche, insbesondere Haut, zeigt. Auch soll eine Anwendung auf Schleimhäuten oder in Körperhöhlen wie z.B. vaginale, u-rethrale, rectale Applikation möglich sein.

### Lösung der Aufgabe

Diese Aufgabe wird erfindungsgemäß durch verschäumbare, vorwiegend transparente, Zubereitungen auf wässriger oder wässrig-alkoholischer Basis gelöst, welche folgende Komponenten aufweisen:
a) ein oder mehrere Kohlenhydrattenside, insbesondere ausgewählt aus
   ai) Zuckerestern von C₁₂-C₁₀ gesättigten, ungesättigten, partial gesättigten Fettsäuren; polyoxyethylierten, bzw. polyoxypropylierten Zuckerestem mittelkettiger und höherkettiger (C8-C18-)Fettsäuren;
   aii) Zuckerethem von C₈-C₂₀ gesättigten, ungesättigten, partial gesättigten Fettalkoholen; polyoxyethylierten, bzw. polyoxypropylierten Zuckerethern;
      oder Mischungen hiervon,
      insbesondere in einer Menge von insgesamt 0,1 bis 20 Gew.%;
b) 0,1 bis 20 Gew. %, bevorzugt 0,1 bis 10 Gew.% und vor allem 1 bis 6 Gew.%, Co-Tenside, vorzugsweise ausgewählt aus
   bi) nichtionischen ethoxylierten/ propoxylierten Tensid-, Fettalkoholen, Fettsäuren, nämlich ethoxylierten und / oder propoxylierten Fettalkoholen, ethoxylierten und / oder propoxylierten Fettsäuren der Kettenlänge C₈ bis C₂₀ mit einem Ethoxylierungs- bzw. Propoxylierungsgrad von 8-40 und mit einem HLB- Wert von 9 bis 20 (insbesondere 9-18); oder Mischungen hiervon;
   bii) Co- Tensiden mit Wirkstoffcharakter (antimikrobielle Eigenschaften)
      bii1) quartären Ammoniumverbindungen,(wie z.B. Benzalkoniumchlorid) in einer Menge von bevorzugt 0,1 bis 6 Gew.%, vor allem 0,1 bis 4 Gew.%, insbesondere 0,2 bis 0,4 Gew.%,
      bii2) quartären (kationischen) polyoxyethylierten, bzw. polyoxypropylierten Zuckerethem (insbesondere 0,2 bis 6 Gew.%, vorzugsweise 1 bis 4%);
      bii3) Guanidin- Abkömmlingen (z.B. Chlorhexidin); insbesondere 0,01 bis 8 Gew. %, vor allem 0,01 bis 2 Gew.%,
      oder Mischungen hiervon.

Die Zubereitungen weisen ferner einwertige C₂ - C₃ -Alkohole, ausgewählt aus Ethanol, Propanol, Isopropanol oder Mischungen hiervon auf, in einer Menge von 0,1 bis 55 Gew.% und vor allem 1 bis 39 Gew. %.

Des Weiteren können auch 0 bis 30 Gew. % Zusatzstoffe, insbesondere 1 bis 30 Gew. %, vor allem 1 bis 15 Gew. % (zu denen auch u. a. Wirkstoffe, z. B. in Mengen von 0 bis 20 Gew. %, insbesondere 0,01 bis 15 Gew. % gehören können), enthalten sein.

Die Zubereitungen werden auf wässriger bzw. wässrig-alkoholischer Basis hergestellt, so dass der Rest der Zusammensetzung Wasser / Wasser-Alkohol Gemisch ist.

Überraschend werden auf diese Weise Zubereitungen erhalten, welche selbst mit hohem Alkoholgehalt dennoch verschäumbar sind und insofern ohne Treibgase beim Austragen z. B. aus Pumpspray-Flaschen mit entsprechenden Adaptern, in Schaumpräparate überführt werden können. Dabei erfolgt überraschenderweise eine spontane Schaumbildung beim Austragen. Der Schaum fällt kontrolliert zusammen, so dass es zu einer vorteilhaften Verteilung der Inhaltsstoffe ohne frühzeitig unerwünschte Fluidisierung kommt. Die erhaltenen Basispräparate sind insbesondere hypoallergen.
Insofern sind auch keine speziellen schaumbildenden Substanzen wie z. B. Natriumlaurylsulfat oder analoge anionische Tenside oder schaumstabilisierende Monoglyceride Komponenten wie insbesondere Glycerinmonooleat erforderlich. Die erfindungsgemäßen Zusammensetzungen sind daher vorzugsweise frei von derartigen Substanzen und insbesondere von Glycerinmonooleat, Monoglyceriden.

### Nähere Erläuterung der Erfindung

Die vorliegende Erfindung betrifft vorwiegend transparente, stabile Lösungen bzw. Dispersionen, welche mittels geeigneter Vorrichtungen ohne Anwendung vom Treibgas verschäumt werden. Grundlage ist dabei Wasser und insbesondere Wasser im Gemisch mit einem oder mehreren einwertigen aliphatischen Alkoholen. In Verbindung mit den angegebenen Zuckertensiden bzw. Kombinationen hiervon mit Co-Tensiden wird somit ein homogenes, stabiles System erhalten, welches auch mit Wirkstoffen und Zusatzstoffen versetzt werden kann, ohne dass die Stabilität beeinträchtigt wird. Insbesondere kann bei Anwesenheit von ausreichenden Anteilen von Alkohol auch auf Konservierungsmittel verzichtet werden. Die erfindungsgemäßen Systeme stellen feindisperse, homogene Flüssigkeiten oder klare, filtrierbare Lösungen dar, die z. B. aus Pumpspray-Flaschen (z.B. wie solche der Firma Airspray®) in einen aus anwendungstechnischer Sicht stabilen Schaum überführbar sind. Sie können auf kosmetischem, dermatologischem und hygienischem Sektor eingesetzt werden, insbesondere auch je nach Wahl von Co-Tensiden. Letztere liegen bevorzugt in einer Menge von 0,1 bis 20 Gew.%, insbesondere 0,1 bis 10 Gew. % oder 1 bis 6 Gew.% vor.

In den erfindungsgemäßen Zubereitungen liegen bevorzugt entweder ein oder mehrere Kohlenhydrattenside ai, aii), oder Mischungen, (z. B. 0,1 bis 20 Gew.%) vor allem Zuckerester von C₁₂-C₁₈ gesättigten, ungesättigten, partial gesättigten Fettsäuren und Zuckerether von C₈-C₂₀ gesättigten, ungesättigten, partial gesättigten Fettalkoholen; insbesondere aber auch Kombinationen aus einem oder mehreren Tensiden a) (ai, aii) zusammen mit einem oder mehreren Tensiden b) (bi,bii1,2,3, insbesondere 0,1 bis 20 Gew.%) vor.

In derartigen Zusammensetzungen können insbesondere 0 bis 60 Gew. %, bevorzugt 1 bis 55 Gew. %, vor allem 1 bis 39 Gew. % eines oder mehrerer einwertiger Alkohole wie beschrieben vorliegen.

Hierin können vor allem auch 1 bis 30 Gew. % Zusatzstoffe wie angegeben enthalten sein.

Bevorzugt weisen die Zusammensetzungen einen oder mehrere Zuckerester, insbesondere in einer Menge von 0,1 bis 10 Gew. %, vor allem C₁₂-C₁₈₋Fettsäure-Zuckerester, insbesondere C₁₆ -C₁₈, auf, welche abgeleitet sind von Glukose, Saccharose. Geeignet sind auch substituierte Derivate der Kohlenhydrate, wie z. B. Alkylsubstituierte Produkte, wie Methylglukose. Ganz besonders bevorzugt ist hier Saccharosestearat / palmitat (wie das unter dem Namen Crodesta® F 110 erhältliche Produkt).

In einer weiteren bevorzugten Ausführungsform werden C₈-C₂₀-Fettalkohol-Zuckerether, bevorzugt in einer Menge von 0,1 bis 10 Gew. %, vor allem 0,3 bis 4 Gew.%, eingesetzt, welche sich von Glukose, Saccharose, Methylglucose ableiten. Ganz besonders bevorzugt ist hier (Decylglucosid) wie das unter dem Namen Plantacare® 2000 UP) erhältliche Produkt.

In einer weiteren Ausführungsform werden Zuckerester, -ether, insbesondere Kohlenhydrate vom Typ Glukose-, Saccharose- C₁₆-C₁₈ -Fettsäure- bzw. C₈-C₂₀₋Fettalkohol-Derivate wie beschrieben mit einem oder mehreren Co- Tensiden der Gruppe bi), insbesondere Macrogolabkömmlingen mit 8-25 EO Gruppen; oder mit quarternären Ammoniumverbindungen (bii1) und/oder Guanidinen (bii3), kombiniert.

Ganz besonders bevorzugt sind hier als Tenside a) Crodesta® F110 (Saccharosestearat/palmitat) und Plantacare® 2000UP (Decylglucosid).

Bevorzugt sind hier 1 bis 55, vor allem 1-39 Gew.%, Alkohol wie beschrieben, oder Alkohol und Zusatzstoffe wie beschrieben, enthalten.

Die Co-Tenside bi) weisen bevorzugt einen HLB- Wert von 9 bis 20, insbesondere 9 bis 18, auf und sind ferner vor allem ausgewählt aus Grundsubstanzen mit einer Kettenlänge von C₈ bis C₂₀ wie z. B. Alkyl-C₁₀-C₁₈-Fettalkoholalkoxylaten, und weisen insbesondere Alkoxylierungsgrade von 8 bis 40, bevorzugt 8 bis 25, auf.

Die Co-Tenside mit Wirkstoffcharakter bii) sind bevorzugt ausgewählt aus einem oder mehreren Di-C₈- bis C₁₄-n-Alkyldimethylammonium- Verbindungen, Mono-n-C₁₀-C₁₄-Alkyldimethylbenzylammonium-Verbindungen, z. B. Chloride, Bromide z. B. Benzethoniumchlorid oder Alkylate wie z. B. Didecylmethylpoly(oxethyl)ammonium-propionat; Guanidinen wie Alkyl (C8-C18)-propylendiaminguanidiniumacetat, Polyhexamethylenbiguanid, Octinidinhydrochlorid, oder Mischungen hiervon.

Auch quartemäre Zuckerverbindungen sind hier geeignet wie insbesondere Lauryl-methyl-Gluceth-10-Hydroxypropyldimmoniumchlorid.

In den erfindungsgemäßen Zubereitungen können 0 bis 30 Gew.%, insbesondere 1 bis 30 Gew.% Zusatzstoffe enthalten sein. Diese können insbesondere - vor allem je nach beabsichtigter Anwendung- ausgewählt sein aus pH- Wert Regulatoren, Konsistenzgebem, Stabilisatoren, Feuchthaltem, Farbstoffen, Geruchskorrigentien (Parfümstoffe), Wirkstoffen, Pflegestoffen, oberflächenaktiven Hilfsstoffen, Konservierungsmitteln oder Mischungen hiervon.

In einer weiteren Ausgestaltung der Erfindung weisen die Zusammensetzungen einen oder mehrere einwertige Alkohole, ausgewählt aus Ethanol, Propanol, Isopropanol oder Mischungen hiervon auf, in einer Menge von 0 bis 60 Gew.%, insbesondere 0,1 bis 55 Gew.%, vor allem 1 bis 45 und ganz besonders 1 bis 39 Gew.%.

In einer weiteren bevorzugten Ausgestaltung der Erfindung weisen die Zusammensetzungen 1 bis 30 Gew.%, vorzugsweise 1 bis 15 Gew.%, Zusatzstoffe, wie erläutert, und 1 bis 55 Gew.%, insbesondere 1 bis 39 Gew.%, eines oder mehrerer einwertiger Alkohole wie beschrieben auf.

In den beschriebenen Zubereitungen werden das oder die Kohlenhydrattenside a) (i,ii, Mischungen) in gleicher, insbesondere geringerer Konzentration als das oder die Co-Tenside b) (i,ii1,2,3, oder Mischungen) eingesetzt, insbesondere beträgt das Verhältnis von a) zu b) 1:1 , vorzugsweise 1:2 bis 1:10. vor allem 1:2 bis 1:6.

Vorteilhafterweise werden die vorgenannten Zuckerester bzw. -ether in Kombination mit einem oder mehreren nichtionischen polyoxyethylierten und/oder polyoxypropylierten Tensiden, insbesondere Fettalkoholen, mit einem HLB Wert von 9 bis 18, (bevorzugte Kombination a) (ai,aii) + bi)) kombiniert. Insbesondere beträgt deren Kettenlänge C₈ bis C₂₀, vor allem C₈ bis C₁₈ . Bevorzugt liegt hier der EO/PO Grad zwischen 8 und 40. Hierzu gehören insbesondere Mulsifan^{®} RT 203/80 = C12-15 Pareth 12, Cremophor^{®} A 25 = Cetomacrogol 25 (Ph.Europ.)

In einer weiteren bevorzugten Ausgestaltung der Erfindung werden ein oder mehrere Zuckerester oder ein oder mehrere Zuckerether oder Mischungen hiervon, insbesondere Glukose- oder Saccharose- C16/18 bzw. C10/14-Derivate, mit Macrogolabkömmlingen als nicht ionische Co- Tenside mit 8-40 EO- Gruppen, oder in einer weiteren Ausführungsform mit nichtionischen Co-Tensiden wie polyoxyethylierte Fettsäureether, z.B. Mulsifan^{®} RT 203/80 (C12-15 Pareth-12), Ceteareth 20 (Eumulgin ® B2) , Cetomacrogol, und mit Zusatzstoffen, insbesondere gewählt aus Feuchthaltern, z. B. Glycerol und / oder Sorbitol, Harnstoff, Wirk- und

Pflegestoffen(u.a. Phospholipide, z.B. Lecithin), Konsistenzgebem, Stabilisatoren, pH- Wert Regulatoren kombiniert.

Ganz besonders bevorzugt sind als Wirkstoffe, insbesondere in vorstehend beschriebenen Kombination a) +bi) ein oder mehrere Wirkstoffe, ausgewählt aus UV-Filtem, Vitaminen, Lecithin, oder Pflanzenextrakten wie z.B. Kamillen-, Amika-, Hamamelis-, Calendulaextrakt, Johanneskraut, oder Antiallergika; oder antiphlogistisch wirksamen Stoffen wie z.B. Indometacin, Diclofenac, Antimykotika, Antiallergika enthalten.

Derartige Zusammensetzungen weisen insbesondere 1 bis 39 Gew.% Ethanol auf.

Diese Zubereitungen sind insbesondere für die kosmetische und dermatologische Anwendung oder als Mittel für den Hygienebereich geeignet. Außer der topischen Applikation sind die Schaumpräparate auch für den Intimbereich und zur Fussbehandlung einsatzfähig.

In einer anderen erfindungsgemäßen Ausführungsform werden Zuckerester oder Zuckerether ai), aii) oder Mischungen wie beschrieben, insbesondere die oben als bevorzugt genannten, mit CoTensiden der Gruppe bii1),bii2), bii3) oder Mischungen hiervon kombiniert. Als Zusatzstoffe (z. B. 1 bis 30 Gew.%) können hier insbesondere pH- Wert Regulatoren, Korrosionsinhibitoren, Konsistenzgeber, Lecithine, Geruchskorrigentien oder Mischungen hiervon vorhanden sein. Sofern Alkohol, bevorzugter Weise, vorliegt, sind der oder die Alkohol(e) insbesondere ausgewählt aus C₂-C₃-Alkoholen wie Ethanol und Isopropanol und in einer Menge von insbesondere 1 bis 39 Gew. % vorhanden.

Geeignete Komponenten der Gruppe bii) sind hier vor allem Di-C₈- bis C₁₄-n-Alkyldimethylammonium- Verbindungen, Mono-n-C₁₀-C₁₄-Alkyldimethylbenzyl-ammonium-Verbindungen, z. B. Chloride, Bromide z. B. Benzethoniumchlorid oder Alkylate wie z. B. Didecylmethylpoly(oxethyl)ammonium-propionat; Guanidine wie Alkyl (C8-C18)-propylendiaminguanidiniumacetat, Polyhexamethylenbiguanid, Octinidinhydrochlorid; oder Mischungen hiervon.

Derartige Zusammensetzungen eignen sich auch zur Behandlung von harten Oberflächen, z.B. als Desinfektions- und Reinigungsmittel für Oberflächen und ärztliche Instrumente, aber auch zur Desinfektion von Haut und Händen, von Menschen.

### Nähere Beschreibung der Inhaltsstoffe

Die einzelnen Inhaltsstoffe werden nachfolgend näher beschrieben:

### I. Kohlenhydrattenside a)

Diese werden ausgewählt aus gesättigten, ungesättigten, partialgesättigten Zuckerestern von C₁₂-C₁₈-Fettsäuren, und/oder gesättigten, ungesättigten, partialgesättigten Zuckerethern von C₈-C₂₀-Fettalkoholen, in einer Menge von insgesamt 0,1 bis 20 Gew.%, insbesondere 0,1 bis 10 Gew.%, insbesondere 0,3 bis 3, und vor allem 0,5 bis 2 Gew.%. Als Kohlenhydrate kommen dabei Mono- Di-und/oder Oligo-Saccharide infrage. Insbesondere bevorzugt sind Glukose, Methylglucose, Fruktose, Saccharose. Besonders bevorzugt sind Glukose und Saccharose.

Als Säuren kommen insbesondere organische aliphatische Carbonsäuren infrage. Hierzu gehören insbesondere Caprylsäure, Caprinsäure, Laurinsäure, Myristinsäure, Palmitinsäure, Stearinsäure, Ölsäure und auch Mischungen dieser. Besonderes bevorzugt sind hier C₁₆-C₁₈-Säuren, welche mit Glucose oder Saccharose verestert sind. Saccharoseester sind vorwiegend Sucrose stearat, - palmitat, -laurat oder -oleat. Ganz besonderes bevorzugt sind C₁₆/C₁₈ Zuckerester, insbesondere in einer Menge von 0,1 bis 10 Gew.%, vor allem Saccharosestearat/ palmitat, z.B. Crodesta® F110 oder F160.

Werden derartige Ester eingesetzt, ist es bevorzugt, eine basische Komponente hinzuzusetzen, welche den Lösevorgang positiv beeinflusst. Eine derartige basische Komponente (pH- Wert Regulator) kann herkömmlich eingesetztes Alkali sein, wie KOH, NaOH, primäre, sekundäre und tertiäre Amine, wie Diethylamin oder Triethylamin oder Diethanolamin oder Triethanolamin. Eine dadurch eventuell bedingte Erhöhung des pH-Wertes des Systems z. B. auf pH 11 kann durch einfache Zugabe entsprechender, insbesondere physiologisch verträglicher Säuren, wie z.B. Milch-, Zitronensäure oder auch Salzsäure auf einen Bereich von 5 bis 7,5 reduziert werden oder falls ein erhöhter pH- Wert für die gewünschte Anwendung geeignet ist, auch beibehalten werden. Dies kann insbesondere bei Flächendesinfektionsmitteln der Fall sein. Die alkalischen Mittel werden in ausreichender, üblicherweise geringer Menge von ca. 1% und kleiner eingesetzt.

Eine weitere Gruppe geeigneter Kohlenhydrattenside stellen die Zuckerether dar. Hierbei handelt es sich um Glucoside aus den vorgenannten Zuckem und ein- oder mehrwertigen Alkoholen mit C₈- bis C₂₀ Kettenlänge. Ganz besonders geeignet sind hier Glukoseether mit einer bevorzugten Kettenlänge im Alkylteil von ca. C₈-C₁₈, vor allem C₁₀C₁₄-Zuckerether, und vor allem die hierzu unter der Namen Plantacare® beschriebenen Zuckerether- Produkte, insbesondere Decylglucosid (Plantacare® 2000 UP). Diese werden bevorzugt in einer Menge von 0,3 bis 4 Gew.% eingesetzt.

Eine weitere Gruppe geeigneter Verbindungen der Kohlenhydrattenside a) sind Umsetzungsprodukte von Zuckern z.B. in Form von Glucose oder Methylglucose, mit mittelkettigen oder höherkettigen, also C₈-C₁₄ bzw. C₁₆-C₂₂ Fettsäuren und /oder Ethylenoxid/ Propylenoxid. Dabei werden ethoxylierte bzw. propoxylierte Zuckerester bzw. ethoxylierte bzw. propoxylierte Zuckerether erhalten. Hierzu gehören z. B. PEG-20 Methylglucose-Sesquistearat, PEG-120. Methlyglucosedioleat bzw. Methyl-Gluceth-10, Methyl-Gluceth-20, PPG-10-Methylglucoseether, PPG-20-Methylglucoseether.

Derartige Substanzen sind z.B. die unter dem Handelsnamen Glucam^{®} bekannten Produkte der Firma Chemron.

Wie erwähnt, können mit den Kohlenhydrattensiden a), insbesondere den Zuckerestem aus Fettsäuren wie beschrieben oder den PEG/PPG- Zuckerethem wie beschrieben anstelle der genannten nicht ionischen Co-Tenside bi) als Co-Tenside auch antimikrobiell wirkende Substanzen der Gruppe bii) wie erläutert, z. B. in Form von kationischen Verbindungen wie Benzalkoniumchlorid, Didecylmethylpoly-(oxethyl)ammonium-propionat bzw. Alkyl (C8-C18)-propylendiamin-guanidiniumacetat oder Chlorhexidin oder auch quaternisierten PEG/PPG Zuckerethem eingesetzt werden.

### II. Co-Tenside b)

Zu geeigneten Co-Tensiden bi) gehören nichtionische polyoxyethylierte und/oder polyoxypropylierte Fettsäuren und fettalkohole mit einer Kettenlänge von C₈ bis C₂₀ und einem Ethoxylierungs- bzw. Propoxylierungsgrad von 8 bis 40, insbesondere 8 bis 25, wobei die Kombination aus Oxylierungsgrad und Kettenlänge jeweils die Wasserlöslichkeit, bzw. die Dispergierbarkeit bedingt. Bevorzugte Gruppen geeigneter nichtionischer Co-Tenside bi) sind ethoxylierte / propoxylierte C₈-C₁₈-Fettsäuren bzw. -Fettalkohole, mit einem Ethoxylierungs-1 Propoxylierungsgrad von 8-25.

Die nichtionischen Co-Tenside der Gruppe bi), liegen bevorzugt in Mengen von 0,1 bis 10, insbesondere 1-6 Gew.% vor.

Bevorzugt weisen diese HLB- Werte von 9-20, insbesondere 9 bis 18, auf.

Weitere besonders bevorzugte Co-Tenside sind solche der Gruppe bii1) wie z.B. quartäre Ammoniumverbindungen, wie Mono- oder Di-C₈- bis C₁₄-n-Alkyl(di)methyl-ammonium-Verbindungen, Mono, Di -n-C₁₀-C₁₄alkyl-(di)methylbenzylammonium-Verbindungen" z.B. Chloride, Bromide z. B. Benzalkoniumchlorid oder Alkylate wie Didecylmethylpoly(oxethyl)-ammoniumpropionat; Guanidine (bii3) wie Alkyl (C8-C18)-propylendiaminguanidinium-acetat, Polyhexamethylenbiguanid, Octenidinhydrochlorid, oder andere Stickstoffverbindungen),wie Cetylpyridiniumchlorid, welche gleichzeitig auch desinfizierenden Wirkstoffcharakter aufweisen. Die Konzentration beträgt in der Regel 0,1 bis 6 Gew.%, vor allem 0,1 bis 4 Gew.%, insbesondere 0,2 bis 0,4 Gew.%, und gewährleistet neben der lösungsbegünstigenden Wirkung eine gute Wirksamkeit u.a. gegen grampositive und gramnegative Bakterien, Pilze und Viren (z.B. HBV-Viren).

Weitere geeignete Co- Tenside der Gruppe bii2) sind Umsetzungsprodukte von Zuckern z.B. in Form von Glucose oder Methylglucose, mit Ethylenoxid/ Propylenoxid und Quatemisierung derartiger ethoxylierter bzw. propoxylierter Zuckerether. Hierzu gehören beispielsweise Lauryl-methyl-Gluceth-10-Hydroxypropyldimmoniumchlorid mit Gluceth = PEG (Methyl)glucose, in Mengen wie vorstehend angegeben.

### III. Zusatzstoffe

Zu geeigneten Zusatzstoffen gehören solche, die für die jeweilige Anwendung (kosmetisch (nicht therapeutisch), hygienisch oder dermatologisch) von Vorteil sind und entsprechend ausgewählt werden können. So können insbesondere eingesetzt werden:
- Feuchthalter wie Glycerol, Sorbitol, Hamstoff;
- Konsistenzmittel wie mehrwertige Alkohole, wie Propylenglykol, Celluloseether, wie Methylcellulose, Hydroxyethylcellulose, Hydroxypropylcellulose, Kolloide wie Guar Gum, Xanthan, Abil®-Typen (=Poloxamer-Polysiloxan-Copolymere);
- Stabilisatoren wie Antioxidantien, EDTA;
- Geruchskorrigentien wie z. B. die nachfolgend genannten ätherischen Wirkstoffe oder synthetische Mittel, wie Parfümstoffe, Linalool, Geraniol oder Rosenöl ;
- Pflegestoffe wie Rückfettende Substanzen (z.B. Cetiol HE), Panthenol (Pantothenylalkohol), Allantoin, Bisabolol;
- Farbstoffe;
- Konservierungsstoffe wie Benzoesäure bzw. deren Salze, Propionsäure bzw. deren Salze, Salicylsäure bzw. deren Salze, Sorbinsäure bzw. deren Salze, 1,3-Butandiol, Benzylalkohol, Phenylethanol, 4-Hydroxybenzoesäuremethyl-, - ethyl-, -propyl-, -butylester,
- pH-Wert Regulatoren, wie KOH oder NaOH, Ammoniak, primäre, sekundäre oder tertiäre Amine oder Alkanolamine oder andere geeignete basische Verbindungen, wie N-Methylglucosamin, Säuren wie Milchsäure, Zitronensäure, Weinsäure, Essigsäure, Salzsäure;
- übliche oberflächenaktive Hilfsstoffe wie nichtionische Tenside: Polysorbate, Aminoxide; oberflächenaktive Hilfsstoffe zur Verbesserung von Produkteigenschaften, insbesondere bei nicht topischer Desinfektionsanwendung wie Carboxylate, Sulfate (Alkylsulfate, Fettalkoholethersulfate, Glycerinethersulfate, Fettsäureethersulfate, Monoglycerid(ether)sulfate, Fettsaureamid(ether)sulfate, Alkyloligoglucosidsulfate), Sulfonate (alkylsulfonate, Alkylbenozolsulfonate, Olefinsulfonate, Alkyleethersulfonate), Phosphate, wie Alkyletherphosphate, Fettsäureisethionate, Fettsäuresarcosinate, Mono- und Dialkylsulfosuccinate, Mono- und Dialkylsulfosuccinamate, Ethercarbonsäure und deren Salze, N-Acylaminsäure (z.B. Acylglutamate, Acyllactylate), Aminseifen, insbesondere Diethanolamin- und Triethanolaminseifen;
- Wirkstoffe wie Vitamine und deren Abkömmlinge, wie beispielsweise Vitamin A, E, C bzw. geeignete Derivate hiervon wie Ester z.B. Palmitat, Acetat oder Phosphat, Vitamin B₆ (Pyridoxin), Vitamin PP (Nicotinsäureamid);
- Wirkstoffe z. B. auf natürlicher Basis wie ätherische Öle und Extrakte aus Pflanzen beispielsweise solche aus Kamille, Calendula, Johanniskraut, Amika, Zaubemuss, Ylang Ylang, Kiefernnadel, Zypresse, Thymian, Minze, Limetten, Orangen, Grapefruit, Mandarine, Wacholder, Baldrian, Zitronenmelisse, Eukalyptus, Thymian, Palmarosa, Rosmarin, Lavendel, Rosenholz, Lemongras, Fichtennadel, Kiefemnadel, Ingwer-, Johannisbeer-, Lindenblüten-, Ringelblumen-, Magnolien-, Algen-, Aloe Vera-, Ananas-, Guave-, Echinacea-, Zimt-, E-feublätterxtrakt oder Mischungen hiervon; Terpene und Terpenoitie wie Campher, Menthol, Cineol oder Mischungen hiervon.
- Weitere Zusatz/ Wirkstoffe sind UV-Filter wie UVB, UVA und Breitbandfilter wie z.B. Ethylhexyl Methoxycinnamat, p-Methoxycinnamat, 4-Methylbenzyliden Campher, Paraaminobenzoesäureester wie N, N-Dimethyl-4-aminobenzoesäure-2-ethylhexylester Homomenthylsacilylat, Octyl Salicylate Octocrylene, Phenylbenzimidazolesulfonic Acid, Benzylidene Malonate Polysiloxane, Benzophenone-3; Methyl Anthranilate, Butyl Methoxydibenzoylmethane, Methylene Bis-Benzotria-zolyt,Tetramethylbutylphenol, Bis-Ethylhexyloxyphenol Methoxyphenyltriazin, Disodium Phenyl Dibenzimidazole Tetrasulfonate, 2-(4-Diethylamino-2-hydroxybenzoyl) Benzoic Acid Hexylester, Endocannabinoide (z.B. N-Palmitoylethanolamin), anorganische UV-Filter wie Zinkoxid und Titandioxid, insbesondere mikronisiert undloder beschichtet.
- Für dermatologische Zwecke eignen sich als Wirkstoffe z. B. Antimykotika, z.B. Clotrimazol, Ciclopiroxolamin oder Ketoconazol, Antiseptika, Antibiotika, z.B. Gentamicin, Hormone bzw. Corticoide z.B. Betamethason sowie deren Ester (propionat, acetat, walerat), Hydrocortison, Methylprednisolon und deren Abkömmlinge, Triamcinolonacetamid, Benzalkoniumchlorid, Chlorhexidin, Dexpanthenol; Ferner geeignet sind Allantoin, Bisabolol sowie Lipoproteine z.B. aus Getreide; Weiter geeignete Wirkstoffe sind Mineralstoffe und Spurenelemente wie Kupfer, Zink, bzw. deren Derivate wie Zincidone® (Zink PCA-Pyrrolidoncarbonsäure), Zinkglukonat oder Kupfergluconat. Weiterhin können adstringierende und sebumregulierende Substanzen eingesetzt werden wie Acnacidor 101 (Propylene Glycol, Hydroxydecanoic Acid), Asebiol® BT (Hydrolyzed Yeast Protein, Pyridoxine, Niacinamide, Glycerin, Panthenol, Propylene Glycol, Allatoin, Biotin), Lipacide® C8C0 (Caproyl Collagen Aminoacids), Sebosoft® (Glycerin, Aqua, Macrogol-8, Caprylyl Glycol, Sebacic Acid, Sodium Polyacrylate), Sepi Control A5 (Capryloylglycine, Methylglycine, Cinnamonum zeylanicum).
   Es können auch kühlende/beruhigende Wirkstoffe wie Frescolat® ML (Menthyl Lactate) oder Eashave® (Sodium Hyaluronate, Wheat Germ Extrakt, Saccharomyces Cerevisiae Extrakt) inkorporiert werden.
   Darüber hinaus sind als Wirkstoffe durchblutungsfördernde Stoffe, z. B. Nikotinsäurederivate wie Methyl- oder Tocopherylnikotinat, Alpha- und Betahydroxysäuren und deren Derivate, z. B. Glykol-, Äpfel-, Zitronen-, Wein-, Milchsäure, Salicylsäure, Isopropylbenzylsalicylate, C12-13 Alkyl Lactate (Cosmacol® ELI) oder auch antiphlogistische und antibakterielle Substanzen wie Triterpene, z. B. Ursolsäure, Glycyrrhizinsäure oder Glycyrrhetinsäure und deren Derivate, z. B. Stearyl Glycyrrhetinate, Kaliumglycyrrhinat; Pantothensäurederivate, z. B. D-Panthenol, Panthenyltriacetat; Allatoin; Bisabolol; Azulene, z. B. Cham-Azulene oder Guaj-Azulen; Phytosphingosine; Triclosan; Chlorhexidin-Derivate und/oder Antischuppenmittel, z. B. Climbazol oder Piroctone Olamine einsetzbar.
   Daneben können in den erfindungsgemäßen Zusammensetzungen auch antioxidativ sowie zellschützend wirkende Stoffe wie Polyphenole, Flavonoide, z. B. Rutin, Ferulasäure und deren Ester oder lsoflavone wie Soja-Isoflavone oder Coenzym Q 10 als wirksame Zusatzstoffe für eingesetzt werden. Zu erwähnen ist auch der antioxidativ wirkende Komplexbildner EDTA. Dabei können je nach beabsichtigtem Wirkungseffekt geeignete Kombinationen an Wirkstoffen zugesetzt werden.
- Weitere Zusatzstoffe sind Pflegestoffe wie Phospholipide wie insbesondere Lecithin (Sojalecithin) sowie Ceramide und Sterole (z.B. Cholesterol), die zur Ausbildung von Vesikeln (Liposomen) eingesetzt werden können.

In nicht kosmetisch/dermatologischen Anwendungsformen, z.B. als Oberflächen- oder Gerätedesinfektionsmittel, können insbesondere Stabilisatoren wie Korrosionsinhibitoren z. B. Benzotriazol, 2-Butin-1,4-diol oder Triethanolamin und/oder die genannten pH-Wert Regulatoren und/oder Geruchskorrigentien als Zusatzstoffe eingesetzt werden.

### Herstellung

Zur Herstellung von erfindungsgemäßen Zubereitungen werden das/die Zuckertenside in Wasser, ggf. erwärmt und gelöst, bei Temperaturen von 20°C bis 90° C, vorzugsweise bei Temperaturen von 40°C bis 90°C, insbesondere 70 bis 90°C.

Anschließend wird das Co-Tensid, bei Temperaturen wie ca. 40°C bis 70°C hinzugefügt und gerührt, wobei temperaturstabile Zusatzstoffe, sofern vorhanden, hinzugesetzt werden können. Falls der Zusatz von Alkali in Form von NaOH oder KOH bzw. einem Amin, wie Triethylamin zweckmäßig ist, wird das Endprodukt - falls erforderlich- durch Zugabe einer geeigneten Säure, wie z.B. Milchsäure oder Essigsäure auf einen gewünschten pH-Wert eingestellt. Nach dem Abkühlen, vorzugsweise auf Raumtemperatur, werden sodann ggf. vorhandene temperaturlabile Komponenten sowie -falls in der Rezeptur aufgeführt- Alkohol zugesetzt.

Die erhaltene Zubereitung ist homogen feindispers, transparent / klar und kann sofort eingesetzt werden, z. B. aus entsprechenden Behältern, insbesondere Pumpspray Flaschen.

### Anwendung

Die erfindungsgemäßen schaumbildenden Systeme zeigen Eigenschaften, welche gegenüber anderen, bekannten Systemen vielfältige Vorteile aufweisen. Insbesondere verfügen sie über eine niedrige allergene Potenz, wobei zudem von einer sehr guten Verträglichkeit ausgegangen werden kann. Somit können diese Zusammensetzungen insbesondere angewendet werden im kosmetischen (nicht therapeutischen), im hygienischen oder auch im pharmazeutischdermatologischen Bereich, insbesondere bei Menschen. Sie können insbesondere angewendet werden auf große Flächen der Haut 1 Kopfhaut oder auch auf harte Oberflächen, z.B. aus Keramik oder Kunst- bzw. Naturstoffen. Je nach weiteren Zusatz-, insbesondere Wirkstoffen können sie vor allem eingesetzt werden zur topischen Anwendung auf Haut als Antimykotika (z.B. bei Pityriasis versicolor), oder als Antitranspirans/Deodorant bei vermehrter Schweißneigung und Geruchsbildung; weiterhin als Antipruriginosum bei generalisiertem Juckreiz (jeweils auch im nicht therapeutischen Bereich); als Antiphlogistikum bei Muskel- oder Gelenkschmerzen; sie können eingesetzt werden als Kosmetikum (nicht therapeutisch) z. B. als Sonnen, After-Sun Präparat, als Anti- Akne- Mittel oder als hydratisierender Pflegeschaum, zum Einsatz bei empfindlicher / Problem-/Kinderhaut sowie im Intimbereich und zur Fußbehandlung, weiterhin als Mittel zur Bekämpfung von Haarausfall (z.B. bei hormonhaltigen Wirkstoffen). Der Einsatz des Mittels auf Haaren ist überraschenderweise auch deshalb unproblematisch, da keine Verklebungsreaktion der Haare und eine rasche Verdunstung eintritt. Femer können die erfindungsgemäßen Zusammensetzungen zum Auftragen von Wirkstoffen und kosmetischen Substanzen auf empfindlicher /Problem- oder Kinderhaut (Gesicht, Intertrigines etc.), sowie zur Anwendung im Intimbereich und zur Fußbehandlung, bei sehr guter lokaler Verträglichkeit eingesetzt werden. Weiterhin geeignet sind erfindungsgemäße Zubereitungen wie erwähnt z.B. als Sonnenschutzmittel oder zur Anwendung bei einer Bartflechte oder als Anti- Akne-Mittel, z. B. enthaltend Benzoylperoxid, Azelainsäure. Durch Zusatz von z. B. Benzalkoniumchlorid, Chlorhexidin o.ä. und Dexpanthenol können zudem akute Entzündungen (z.B. akute Kontaktdermatitis, Erysipel = Wundrose) oder Verbrennungen (z.B. Erythema solare = Sonnenbrand, evtl. diesbezüglich Zusatz eines Steroids und Menthol) behandelt werden.

Ebenso kann das Mittel als Desinfektionsmittel im Sinne der Keimreduktion sowohl auf der Haut, wie auch im Sinne der Haut- Hände- und (Ober)Flächendesinfektion oder Desinfektion von ärztlichen Instrumenten eingesetzt werden.

Das Mittel kann je nach Anwendungszweck durch Aufsprühen ein- oder mehrmals am Tag auf die Hautfläche bzw. je nach Bedarf auf die zu behandelnde Fläche aufgetragen und verteilt werden, z. B. durch Einreiben.

In Abhängigkeit von der Indikation kann der Alkoholanteil in der Grundlage vorzugsweise angepasst werden.

Wie hieraus ersichtlich ist, gelingt mit den neuartigen Systemen durch einen in der Regel relativ geringen Gehalt eines Kohlenhydrat-Tensids, ggf. in Verbindung mit einem Co-Tensid, z. B. aus der Reihe der nichtionischen Verbindungen oder in Form von quartären Ammoniumverbindungen die Herstellung von Grundlagen, welche für die begrenzte und/oder großflächige Anwendung auf der Haut und auf harten Flächen geeignet ist, insbesondere aufgrund des hypoallergenen Charakters der Basisformulierung. Je nach weiterem Wirkstoff kann die Anwendung dabei den spezifischen Bedürfnissen angepasst werden. Der Substanzauftrag erfolgt dabei sowohl ohne als auch mit Alkohol in Form eines Schaumes ohne Einsatz von Treibgas, insbesondere auch ohne Zusatz schaumbildender anionischer Tenside wie Laurylsulfat etc. oder vor allem auch ohne Einsatz schaumstabilisierender Monoglyceride, wie insbesondere Glycerylmonooleat, (oder -caprylat) d.h. unter Vermeidung der damit verbundenen Nachteile, wobei der Schaum ohne vorzeitige Fluidisierung kontrolliert kollabiert und so ein gleichmäßiges Verteilen und gegebenenfalls Einreiben ermöglicht. Insbesondere kann auch bei Anwesenheit von jeweils ausreichenden Anteilen an Alkohol auf Konservierungsmittel verzichtet werden.

### Beispiele

Die folgenden Beispiele verdeutlichen vorliegende Erfindungen. Die Angaben beziehen sich auf Gewichts- %.

### Beispiele 1 bis 13

Die Zusammensetzungen gemäß den nachfolgenden Beispielen 1 bis 13 wurden wie folgt hergestellt: Das Tensid a) wird in Wasser bei ca. 70° bis 90° C dispergiert. Daraufhin wird das zweite Tensid b) hinzugefügt und ca. 10 Minuten unter Rühren bei 80° C gehalten. Es entsteht eine klare Lösung. Die Base, sofem erwünscht, wird sodann unter Rühren hinzugefügt. Nach dem Abkühlen unter kontinuierlichen Rühren wird bei Raumtemperatur der gegebenenfalls vorhandene Alkohol zugesetzt und ca. 5 Min. gerührt. Sodann erfolgt, falls höhere (pH>8) erhalten und anwendungstechnisch nicht gewünscht werden, eine pH Wert Einstellung mit der angegebenen Säure auf etwa 6,5. Anschließend wird filtriert und in geeignete Austragungsbehälter wie z. B. Pumpspray-Behälter gefüllt.

### Beispiele 11 bis 17

Das Tensid a) wird in Wasser bei ca. 30-50°C gelöst. Daraufhin wird das zweite Tensid b) hinzugefügt und ca. 10 Minuten unter Rühren bei 40-50°C gehalten. Es entsteht eine klare Lösung. Die Lösung wird unter Rühren abgekühlt und bei Raumtemperatur wird Ethanol zugefügt und ca. 5 Min. gerührt. Sodann wird ggf. Milchsäure ad pH 5,5 zugefügt. Anschließend wird die Lösung filtriert und in geeignete Behälter gefüllt.

In den Beispielen bedeuten:
Crodesta^{®} F110: Saccharosestearat/ palmitat (Tensid a)
Plantacare^{®} 2000UP: Decylglucosid (Tensid a)
Glucam E 20: Methyl Gluceth- 20 (Tensid a)
BAC 50%: Benzalkoniumchlorid (Co- Tensid bii1)
Mulsifan RT 203/80: C12-15-Pareth 12 (Tensid bi)
Eumulgin B2: Ceteareth-20 (Tensid bi)
Cremophor A25: Ceteareth-25 (Co- Tensid bi)

**Tabelle 1**

| Beispiel / Inhaltsstoff | 1 | 2 | 3 | 4 | 5 |
|---|---|---|---|---|---|
| Crodesta F 110 a) | 0,5 | 0,5 | 0,5 | 0,5 | 0,5 |
| BAC 50% bii1) | 3,0 | - | - | - | - |
| Mulsifan RT 203/80 bi) | - | 4,0 | 4,0 | - | 4,0 |
| Plantacare® 2000 UP,a) | - | - | - | 4,0 | - |
| Ethanol | 25 | - | - | 5,0 | 5,0 |
| Sorbitol | - | - | 6,0 | - | - |
| Phenylethylalkohol | - | - | - | - | 0,5 |
| Triethylamin | 0,5 | - | - | - | - |
| 5% KOH | - | q.s. | q.s. | q.s. | q.s. |
| Sorbinsäure | - | 0,1 | 0,1 | - | - |
| Essigsäure | q.s. ad pH 6,5 | - | - | - | - |
| Milchsäure | - | - | - | q.s. ad pH 6,5 | - |
| Propylparaben | - | 0,04 | 0,04 | - | - |
| Methylparaben | - | 0,1 | 0,1 | - | - |
| Wasser | Ad 100 | Ad 100 | Ad 100 | Ad 100 | Ad 100 |

**Tabelle 2**

| Beispiel / Inhaltsstoff | 6 | 7 |
|---|---|---|
| Crodesta F 110 (a) | 0,5 | 0,5 |
| Cremophor A25 (bi) | 4,0 | - |
| Mulsifan RT203/80 (bi) | - | 4,0 |
| Ethanol | 5,0 | 25 |
| Triethylamin | 0,5 | - |
| 5% KOH | - | q.s. |
| Salzsäure | - | - |
| essigsäure | q.s. ad | - |
| Milchsäure | - | q.s. ad pH 6,5 |
| Zitronensäure | - | - |
| Wasser | Ad 100,0 | Ad 100,0 |

**Tabelle 3**

| Beispiel / Inhaltsstoff | 8 | 9 | 10 |
|---|---|---|---|
| Crodesta F 110 (a) | 0,5 | 0,5 | 0,5 |
| Plantacare ® UP 2000 (a) | 4, 0 4,0 | - | - |
| Mulsifan RT 203/80 (b) | - | 4,0 | 4,0 |
| Ethanol | 25,0 | 7,5 | 25 |
| Propylenglykol | - | 7,5 | - |
| Diethanolamin | - | - | - |
| 5% KOH | q.s. | q.s. | q.s. |
| Salzsäure | - | - | - |
| Essigsäure | - | - | - |
| Milchsäure | q.s. ad pH6,5 | q.s. ad pH6,5 | q.s. ad pH6,5 |
| Wasser | Ad 100,0 | Ad 100,0 | Ad 100,0 |

**Tabelle 4**

| Beispiel / Inhaltsstoff | 11 | 12 | 13 | 14 | 15 | 16 | 17 |
|---|---|---|---|---|---|---|---|
| Plantacare® 2000 UP | 0,6 | 0,6 | 0,6 | 4,0 | 0,6 | 0,6 | - |
| Eumulgin B2(bi) | 4,0 | 4,0 | - | 0,4 | 4,0 | 4,0 | 4,0 |
| Glucam E 20 Glucam E 20 (a) | - | - | - | - | - | - | 0,6 |
| Cremophor A25(bi) | - | - | 4,0 | - | - | - | - |
| BAC 50% (bii1) - | - | 0,4 | 0,4 | - | - | - | - |
| Ethanol | 39,0 | 39,0 | 25,0 | 25,0 | 39,0 | 25,0 | 39,0 |
| Sorbitol | - | - | - | - | 4,0 | - | - |
| Panthenol | - | - | - | - | - | 4,5 | - |
| Bethamethasonvalerat | - | - | - | - | - | - | 0,1 |
| Milchsäure | q.s. ad pH 5,5 | q.s. ad pH 5,5 | q.s. ad pH 5,5 | q.s. ad pH 5,5 | q.s. ad pH 5,5 | q.s. ad pH 5,5 | q.s. ad pH 5,5 |
| Wasser | Ad 100,0 | Ad 100,0 | Ad 100,0 | Ad 100,0 | Ad 100,0 | Ad 100,0 | Ad 100,0 |

## Patentansprüche

1. Treibgasfreie verschäumbare Zubereitung auf wässriger oder wässrig-alkoholischer Basis, enthaltend:
a) ein oder mehrere Kohlenhydrattenside, ausgewählt aus
ai) Zuckerestem von C₁₂-C₁₈ gesättigten, ungesättigten, partial gesättigten Fettsäuren; polyoxyethylierten bzw. polyoxypropylierten Zuckerestem mittelkettiger und höherkettiger (C8-C18-)Fettsäuren;
aii) Zuckerethem von C₈-G₂₀ gesättigten, ungesättigten, partial gesättigten Fettalkoholen; polyoxyethylierten bzw. polyoxypropylierten Zuckerethem;
oder Mischungen hiervon, in einer Menge von insgesamt 0,1 bis 20 Gew.%;
b) 0,1 bis 20 Gew. %, Co-Tenside, ausgewählt aus
bi) nichtionischen ethoxylierten und / oder propoxylierten Fettalkoholen, ethoxylierten und / oder propoxylierten Fettsäuren der Kettenlänge C₈ bis C₂₀ mit einem Ethoxylierungs- bzw. Propoxylierungsgrad von 8-40 und mit einem HLB- Wert von 9 bis 20; oder Mischungen hiervon.
bii) Co-Tensiden mit antimikrobielle Eigenschaften, ausgewählt aus
bii1) 0,1 bis 6 Gew.% quartemären Ammoniumverbindungen;
bii2) 0,2 bis 6 Gew.% kationischen quartemären polyoxyethylierten, bzw. polyoxypropylierten Zuckerethem und
bii3)0,01 bis 8 Gew.% Guanidin- Abkömmlingen;
oder Mischungen hiervon,
sowie 0,1- 55 Gew.% eines oder mehrerer einwertiger C₂ - C₃ -Alkohole, ausgewählt aus Ethanol, Propanol, Isopropanol oder Mischungen hiervon.

2. Zubereitungen gemäß Anspruch 1, **dadurch gekennzeichnet, dass** sie 1 bis 30 Gew. % Zusatzstoffe aufweisen.

3. Zubereitungen nach Anspruch 2, **dadurch gekennzeichnet, dass** der oder die Zusatzstoffe ausgewählt sind aus pH- Wert Regulatoren, Konsistenzgebern, Stabilisatoren, Feuchthaltern, Farbstoffen, Pflegestoffen, Wirkstoffen, Geruchskorrigentien, Konservierungsmitteln, oberflächenaktiven Hilfsstoffen oder Mischungen hiervon.

4. Zubereitungen gemäß einem der Ansprüche 1 bis 3, **dadurch gekennzeichnet, dass** sie 1 bis 39 Gew. % eines oder mehrerer einwertiger Alkohole, ausgewählt aus Ethanol, Propanol, Isopropanol oder Mischungen hiervon aufweisen.

5. Zubereitungen gemäß einem der Ansprüche 1 bis 4, **dadurch gekennzeichnet, dass** als Tensid ai) hiervon ein oder mehrere Zuckerester, in einer Menge von 0,1 bis 10 Gew.%, insbesondere C₁₆-C₁₈ Zuckerester, welche abgeleitet sind von Glukose, Methylglucose, Saccharose, enthalten sind.

6. Zubereitungen gemäß einem der Ansprüche 1 bis 4, **dadurch gekennzeichnet, dass** als Tensid aii) ein oder mehrere C₈-C₂₀ Zuckerether, in einer Menge von 0,3 bis 4 Gew.%, enthalten sind, welche sich von Glukose, Methylglucose, Saccharose ableiten.

7. Zubereitungen gemäß einem der Ansprüche 1 bis 6, **dadurch gekennzeichnet, dass** der oder die Zuckerester bzw. -ether a) in Kombination mit einem oder mehreren nichtionischen ethoxylierten und / oder propoxylierten C₈ bis C₂₀ - Fettalkoholen bi) mit einem HLB Wert von vorzugsweise 9 bis 18 kombiniert sind.

8. Zubereitungen gemäß einem der Ansprüche 1 bis 7, **dadurch gekennzeichnet, dass** ein oder mehrere Zuckerester, ein oder mehrere Zuckerether oder Mischungen hiervon mit nichtionischen Tensiden wie C12-15 Pareth 12, Ceteareth 20, Cetomacrogol und Zusatzstoffe, ausgewählt aus Feuchthaltem, Wirk- und Pflegestoffen, Konsistenzgebern, Stabilisatoren, pH-Wert Regulatoren oder Mischungen hiervon enthalten sind.

9. Zubereitungen nach Anspruch 8, **dadurch gekennzeichnet, dass** als Zuckerester bzw- ether - Kohlenhydrate vom Typ der Glukose oder Saccharose - C16/18-Fettsäure- bzw. - C8-20-Fettalkohol-Derivate und als nichtionisches Co-Tensid ein Macrogolabkömmling mit 8-25 EO-Gruppen enthalten ist.

10. Zubereitungen gemäß Anspruch 8 oder 9, **dadurch gekennzeichnet, dass** weiterhin Ethanol, Propanol, Isopropanol oder Mischungen hiervon in einer Menge von 1 bis 55 Gew.% enthalten sind.

11. Zubereitungen gemäß einem der Ansprüche 1 bis 10, **dadurch gekennzeichnet, dass** ein oder mehrere Zuckerester oder ein oder mehrere Zuckerether ai, aii) oder Mischungen hiervon mit quarternären Ammoniumverbindungen bii1), Guanidinen bii3) oder Mischungen hiervon kombiniert sind.

12. Zubereitungen nach Anspruch 11, **dadurch gekennzeichnet, dass** weiterhin 1 bis 39 Gew.% eines oder mehrerer Alkohole, ausgewählt aus Ethanol, Propanol, Isopropanol oder Mischungen hiervon, und 1 bis 30 Gew.% Zusatzstoffe, ausgewählt aus Korrosionsinhibitoren, pH Wert Regulatoren, Geruchskorrigentien, Lecithinen oder Mischungen hiervon enthalten sind.

13. Zubereitungen nach Anspruch 11 oder 12, **dadurch gekennzeichnet, dass** die Co-tenside bii) ausgewählt sind aus Di-C₈- bis C₁₄-n-Alkyldimethylammonium-Verbindungen, Mono-n-C₁₀-C₁₄-Alkyldimethylbenzylammonium-Verbindungen, Alkyl (C8-C18)-propylendiaminguanidiniumacetat, Polyhexamethylenbiguanid, Octinidinhydrochlorid, oder Mischungen hiervon.

14. Zubereitungen gemäß einem der Ansprüche 2 bis 13 , **dadurch gekennzeichnet, dass** als Wirkstoffe ein oder mehrere Wirkstoffe ausgewählt aus UV-Filtern, einem oder mehreren Vitaminen, Lecithin, oder Pflanzenextrakten wie z.B. Kamillen-, Amika-, Hamamelis-, Johanneskraut-, Calendulaextrakt, oder Antiallergika enthalten sind.

15. Verfahren zur Herstellung von Zubereitungen gemäß einem der Ansprüche 1 bis 14, **dadurch gekennzeichnet, dass** man das oder die Zuckertenside (ai, aii) in Wasser, ggf. sofern erforderlich, unter Zugabe von Basen löst und erwärmt auf Temperaturen von 20 - etwa 90°C , sodann bei reduzierter Temperatur wie ca. 40°C bis 70°C ggf. das oder die Co- Tenside (bi, bii1,2,3) hinzufügt und rührt, wobei man temperaturstabile Zusatzstoffe, sofern vorhanden, hinzusetzt und nach dem Abkühlen, vorzugsweise auf Raumtemperatur oder darunter, die weiteren, insbesondere temperaturlabilen wie brennbaren Zusatzstoffe wie Alkohol, sofern vorhanden, zusetzt.

16. Verwendung von Zubereitungen gemäß einem der Ansprüche 1 bis 18 als nicht therapeutisches kosmetisches Mittel oder als Mittel für den Hygienebereich.

17. Verwendung gemäß Anspruch 16 als Sonnenschutzpräparat, After Sun-Präparat, Antitranspirans oder Feuchtigkeitsschaum, als Anti- Akne- Mittel, als hydratisierender Pflegeschaum, als Mittel zum Einsatz bei empfindlicher /Problem-/Kinderhaut sowie im Intimbereich und zur Fußbehandlung.

18. Verwendung gemäß Anspruch 16 zur Desinfektion von Haut, Händen, von harten Oberflächen oder ärztlichen Instrumenten.

19. Verwendung von Zubereitungen gemäß einem der Ansprüche 1 bis 15 zur Herstellung eines dermatologisch - pharmazeutischen Mittels zur Anwendung auf Haut, Schleimhaut von Menschen.

20. Verwendung gemäß Anspruch 19 als Antimykotikum, Antiphlogistikum, Anti-Akne- Mittel, als Mittel zur Behandlung von Haarausfall, zur Anwendung bei empfindlicher / Problemhaut oder Kinderhaut, sowie zur Anwendung im Intimbereich und zur Fußbehandlung.

## Claims

1. A propellant gas-free foamable preparation on an aqueous or aqueous-alcoholic basis, containing:
a) one or more carbohydrate surfactants selected from
ai) sugar esters of C₁₂-C₁₈ saturated, unsaturated, partially saturated fatty acids; polyethoxylated or polypropoxylated sugar esters of medium-chain and higher (C₈-C₁₈) fatty acids;
aii) sugar ethers of C₈-C₂₀ saturated, unsaturated, partially saturated fatty alcohols; polyethoxylated or polypropoxylated sugar ethers;
or mixtures thereof, in a total quantity of 0.1 to 20 wt.%;
b) 0.1 to 20 wt.% of co-surfactants selected from
bi) nonionic ethoxylated and/or propoxylated fatty alcohols, ethoxylated and/or propoxylated fatty acids of a chain length of C₈ to C₂₀ with a degree of ethoxylation or propoxylation of 8-40 and with an HLB value of 9 to 20; or mixtures thereof,
bii) co-surfactants with antimicrobial properties selected from
bii1) 0.1 to 6 wt.% of quaternary ammonium compounds;
bii2) 0.2 to 6 wt.% of cationic quaternary polyethoxylated or polypropoxylated sugar ethers and
bii3) 0.01 to 8 wt.% of guanidine derivatives;
or mixtures thereof,
together with 0.1-55 wt.% of one or more monohydric C₂-C₃ alcohols selected from ethanol, propanol, isopropanol or mixtures thereof.

2. Preparations according to claim 1, **characterised in that** they comprise 1 to 30 wt.% of additives.

3. Preparations according to claim 2, **characterised in that** the additive(s) is/are selected from pH value regulators, consistency providers, stabilisers, moisture retainers, dyes, conditioners, active ingredients, odour-correcting agents, preservatives, surface-active auxiliary substances or mixtures thereof.

4. Preparations according to any one of claims 1 to 3, **characterised in that** they comprise from 1 to 39 wt.% of one or more monohydric alcohols selected from ethanol, propanol, isopropanol or mixtures thereof.

5. Preparations according to any one of claims 1 to 4, **characterised in that** one or more sugar esters, in particular C₁₆-C₁₈ sugar esters which are derived from glucose, methyl glucose, sucrose is/are present in a quantity of 0.1 to 10 wt.% as surfactant ai).

6. Preparations according to any one of claims 1 to 4, **characterised in that** one or more C₈-C₂₀ sugar ethers which are derived from glucose, methyl glucose, sucrose is/are present in a quantity of 0.3 to 4 wt.% as surfactant aii).

7. Preparations according to any one of claims 1 to 6, **characterised in that** the sugar ester(s) or ether(s) a) are combined in combination with one or more nonionic ethoxylated and/or propoxylated C₈ to C₂₀ fatty alcohols bi) with an HLB value of preferably 9 to 18.

8. Preparations according to any one of claims 1 to 7, **characterised in that** one or more sugar esters, one or more sugar ethers or mixtures thereof are present with nonionic surfactants such as C12-15 pareth 12, ceteareth 20, cetomacrogol and additives selected from moisture retainers, active substances and conditioners, consistency providers, stabilisers, pH value regulators or mixtures thereof.

9. Preparations according to claim 8, **characterised in that** carbohydrates of the glucose or sucrose C16/18 fatty acid or C8-20 fatty alcohol derivative type are present as the sugar esters or ethers and a macrogol derivative with 8-25 EO groups is present as a nonionic co-surfactant.

10. Preparations according to claim 8 or claim 9, **characterised in that** ethanol, propanol, isopropanol or mixtures thereof are furthermore present in a quantity of 1 to 55 wt.%.

11. Preparations according to any one of claims 1 to 10, **characterised in that** one or more sugar esters or one or more sugar ethers ai), aii) or mixtures thereof are combined with quaternary ammonium compounds bii1), guanidines bii3) or mixtures thereof.

12. Preparations according to claim 11, **characterised in that** 1 to 39 wt.% of one or more alcohols selected from ethanol, propanol, isopropanol or mixtures thereof, and 1 to 30 wt.% of additives selected from corrosion inhibitors, pH value regulators, odour-correcting agents, lecithins or mixtures thereof are furthermore present.

13. Preparations according to claim 11 or claim 12, **characterised in that** the co-surfactants bii) are selected from di-C₈-C₁₄-n-alkyldimethylammonium compounds, mono-n-C₁₀-C₁₄-alkyldimethylbenzylammonium compounds, alkyl-(C₈-C₁₈)-propylenediamineguanidinium acetate, polyhexamethylene biguanide, octenidine hydrochloride or mixtures thereof.

14. Preparations according to any one of claims 2 to 13, **characterised in that** one or more active ingredients selected from UV filters, one or more vitamins, lecithin, or plant extracts such as for example chamomile, arnica, witch hazel, hypericum, calendula extract, or antiallergic agents are present as active ingredients.

15. A method for producing preparations according to any one of claims 1 to 14, **characterised in that** the sugar surfactant(s) (ai, aii) are dissolved in water, optionally where necessary with addition of bases, and heated to temperatures of 20 to approx. 90°C, then the co-surfactant(s) (bi, bii1,2,3) is/are optionally added at reduced temperature such as approx. 40°C to 70°C and stirred, wherein temperature-stable additives, where present, are added and after cooling, preferably to room temperature or below, the further, in particular temperature-labile and flammable additives, such as alcohol, if present, are added.

16. Use of preparations according to any one of claims 1 to 18 as a non-therapeutic cosmetic agent or as an agent for hygiene applications.

17. Use according to claim 16 as a sunscreen preparation, after-sun preparation, antiperspirant or moisture foam, as an anti-acne agent, as a hydrating conditioning foam, as an agent for use on sensitive/problem/children's skin and in intimate areas and for foot treatment.

18. Use according to claim 16 for disinfecting skin, hands, hard surfaces or medical instruments.

19. Use of preparations according to any one of claims 1 to 15 for producing a dermatological-pharmaceutical agent for use on human skin and mucosa.

20. Use according to claim 19 as an antimycotic, antiinflammatory agent, anti-acne agent, as an agent for treating hair loss, for use on sensitive/problem skin or children's skin, and for use in intimate areas and for foot treatment.

## Revendications

1. Préparation moussante exempte de gaz propulseur à base d'eau
ou d'eau-alcool, contenant
a) un ou plusieurs tension-actifs hydrocarbonés choisis parmi
ai) les esters de sucre et d'acides gras saturés, insaturés, partiellement saturés en C₁₂ à C₁₈ ; les esters de sucre polyoxyéthylés ou polyoxypropylés et d'acides gras en C₈ à C₁₈ à chaîne moyenne et supérieure;
aii) les éthers de sucre et d'alcools gras saturés, insaturés, partiellement saturés en C₈ à C₂₀ ; les éthers de sucre polyoxyéthylés ou polyoxypropylés
ou des mélanges de ceux-ci, en une quantité de 0,1 à 20% en poids au total
b) 0,1 à 20% en poids de co-tensio-actifs, choisis parmi
bi) les alcools gras éthoxylés et/ou propoxylés, non-ioniques, les acides gras éthoxylés et/ou propoxylés de longueur de chaîne en C₈ à C₂₀ avec un degré d'éthoxylation ou de propoxylation de 8 à 40 et avec une valeur HLB (équilibre hydrophile lipophile) de 9 à 20; ou des mélanges de ceux-ci;
bii) les co-tensio-actifs ayant des propriétés anti-microbiennes, choisis parmi
bii1) 0,1 à 6% en poids de composés d'ammonium quaternaires;
bii2) 0,2 à 6% en poids d'éthers de sucre polyoxyéthylés ou polyoxypropylés quaternaires cationiques et
bii3) 0,01 à 8% de dérivés de guanidine;
ou des mélanges de ceux-ci,
ainsi que 0,1 à 55% en poids d'un ou plusieurs alcools monovalents en C₂ à C₃ choisis parmi l'éthanol, le propanol, l'isopropanol ou des mélanges de ceux-ci.

2. Préparations selon la revendication 1, **caractérisées en ce qu'**elles présentent 1 à 30% en poids d'additifs.

3. Préparations selon la revendication 2, **caractérisées en ce que** le ou les additifs sont choisis parmi les régulateurs de pH, les agents donnant de la consistance, les stabilisants, les humectants, les colorants, les substances de soin, les substances actives, les agents correcteurs olfactifs, les conservateurs, les adjuvants tensio-actifs ou des mélanges de ceux-ci.

4. Préparations selon l'une quelconque des revendications 1 à 3, **caractérisées en ce qu'**elles présentent 1 à 39% en poids d'un ou plusieurs alcools monovalents choisis parmi l'éthanol, le propanol, l'isopropanol et des mélanges de ceux-ci.

5. Préparations selon l'une quelconque des revendications 1 à 4, **caractérisées en ce que**, en tant que tensioactif de ai), un ou plusieurs esters de sucre sont contenus en une quantité de 0,1 à 10% en poids, en particulier des esters de sucre en C₁₆ à C₁₈, qui sont dérivés du glucose, du méthylglucose, du saccharose.

6. Préparations selon l'une quelconque des revendications 1 à 4, **caractérisées en ce que**, en tant que tensioactif aii), un ou plusieurs éthers de sucre en C₈ à C₂₀ sont contenus en une quantité de 0,3 à 4% en poids, qui sont dérivés du glucose, du méthylglucose et du saccharose.

7. Préparations selon l'une quelconque des revendications 1 à 6, **caractérisées en ce que** le ou les esters ou éthers de sucre de a) en combinaison avec un ou plusieurs alcools gras éthoxylés et/ou propoxylés non ioniques en C₈ à C₂₀ de bi) ayant une valeur HLB de préférence de 9 à 18, sont combinés.

8. Préparations selon l'une quelconque des revendications 1 à 7, **caractérisées en ce qu'**un ou plusieurs esters de sucre, un ou plusieurs éthers de sucre ou des mélanges de ceux-ci, sont contenus avec des tensioactifs non ioniques comme Pareth 12 en C₁₂ à C₁₅, Ceteareth 20, Cetomacrogol et avec des additifs, choisis parmi les humectants, les substances actives et de soin, les agents donnant de la consistance, les stabilisants, les régulateurs de pH ou des mélanges de ceux-ci.

9. Préparations selon la revendication 8, **caractérisées en ce que**, en tant qu'esters de sucre ou éthers de sucre - des glucides du type du glucose ou du saccharose - dérivés d'acides gras en C₁₆/₁₈ ou d'alcools gras en C₈ à C₂₀ et un dérivé de Macrogol ayant de 8 à 25 groupes OE en tant que co-tensioactif non ionique, sont contenus.

10. Préparations selon la revendication 8 ou 9, **caractérisées en ce que** de l'éthanol, du propanol, de l'isopropanol ou des mélanges de ceux-ci sont contenus en outre en une quantité de 1 à 55% en poids.

11. Préparations selon l'une quelconque des revendications 1 à 10, **caractérisées en ce qu'**un ou plusieurs esters de sucre ou un ou plusieurs éthers de sucre ai, aii) ou des mélanges de ceux-ci, sont combinés avec des composés d'ammonium quaternaire bii1), des guanidines bi3) ou des mélanges de ceux-ci.

12. Préparations selon la revendication 11, **caractérisées en ce qu'**en outre, 1 à 39% en poids d'un ou plusieurs alcools choisis parmi l'éthanol, le propanol, l'isopropanol ou des mélanges de ceux-ci, et 1 à 30% en poids d'additifs, choisis parmi les inhibiteurs de corrosion, les régulateurs de pH, les agents correcteurs olfactifs, les lécithines ou des mélanges de ceux-ci, sont contenus.

13. Préparations selon la revendication 11 ou 12, **caractérisées en ce que** les co-tensioactifs bii) sont choisis parmi les composés di-(n-alkyle en C₈ à C₁₄)-diméthylammonium, les composés mono-(n-alkyle en C₁₀ à C₁₄)-diméthylbenzylammonium, l'acétate de (alkyle en C₈ à C₁₈)-propylènediamine-guanidinium, le polyhexaméthylènebiguanide, le chlorydrate d'octinidine, ou des mélanges de ceux-ci.

14. Préparations selon l'une quelconque des revendications 2 à 13, **caractérisées en ce qu'**une ou plusieurs substances actives choisies parmi les filtres UV, une ou plusieurs vitamines, la lécithine, ou des extraits végétaux, comme par exemple l'extrait de camomille, l'extrait *d'amica montana,* l'extrait d'hamamélis, l'extrait de millepertuis, l'extrait de souci *(calendula officinalis),* ou des agents anti-allergiques, sont contenus en tant que substances actives.

15. Procédé pour préparer des préparations selon l'une quelconque des revendications 1 à 14, **caractérisé en ce que** l'on dissout le ou les tensio-actifs de sucre (ai, aii) dans de l'eau, éventuellement dans la mesure où ceci est nécessaire, en ajoutant des bases, et on chauffe à des températures de 20 - environ 90°C, puis on ajoute et on agite éventuellement le ou les co-tensioactifs (bi, bii1,2,3) à une température réduite comme de 40° à 70°C environ, en ajoutant des additifs stables en température, dans la mesure où ils sont disponibles, et après refroidissement, on ajoute les autres additifs en particulier labiles en température ainsi que les additifs combustibles comme l'alcool, dans la mesure où ils sont disponibles, de préférence à la température ambiante, ou à une température inférieure à celle-ci.

16. Utilisation de préparations selon l'une quelconque des revendications 1 à 18, en tant qu'agent cosmétique non thérapeutique, ou en tant qu'agent pour le domaine de l'hygiène.

17. Utilisation selon la revendication 16, en tant que préparation pour la protection solaire, préparation après-soleil, agent anti-transpirant ou mousse hydratante, en tant qu'agent anti-acné, en tant que mousse de soin hydratante, en tant qu'agent à mettre en oeuvre dans le cas de peau sensible/ à problèmes/enfantine, ainsi que dans le domaine intime et pour le traitement podologique.

18. Utilisation selon la revendication 16, pour désinfecter la peau, les mains, les surfaces dures ou les instruments médicaux.

19. Utilisation de préparations selon l'une quelconque des revendications 1 à 15 pour préparer un agent dermatopharmaceutique destiné à être appliqué sur la peau, la muqueuse chez les humains.

20. Utilisation selon la revendication 19 en tant qu'agent antimycosique, anti-inflammatoire, anti-acné, en tant qu'agent pour traiter la chute des cheveux, pour application dans le cas de peau sensible/à problèmes/enfantine, ainsi que pour l'application dans le domaine intime, et pour le traitement podologique.
